# EUROPEAN PATENT APPLICATION

(11) **EP 3 824 938 A1**
(43) Date of publication of application: **26.05.2021**
(21) Application number: 18927045.7
(22) Date of filing: 19.07.2018
(51) Int. Cl.: A61M 25/09

(54) **GUIDE WIRE AND GUIDE WIRE MANUFACTURING METHOD**

(71) Applicant: ASAHI INTECC CO., LTD., Seto-shi, Aichi 489-0071 (JP)
(72) Inventor: YONEZAWA, Satoshi, Aichi 4890071 (JP)
(74) Representative: Prinz & Partner mbB
(86) International application number: PCT/JP2018/027068
(87) International publication number: WO 2020/016986

(57) **Abstract**

A guide wire includes a first core shaft, a second core shaft, a covering member, and joint layer. The first core shaft has a first large-diameter portion and a first small-diameter portion, and the second core shaft has a second large-diameter portion and a second small-diameter portion. The covering member covers a contact portion where the first and second small-diameter portions are arranged oppositely to each other, and at least a part of each of the first and second small-diameter portions adjacent to the contact portion. The joint layer joins the covering member with the first and second small-diameter portions inside the covering member. A length of the covering member is shorter than a sum of the lengths of the first and the second small-diameter portion in the axial direction, and longer than the shorter of the lengths of the first and second small-diameter portions in the axial direction.

## Description

### TECHNICAL FIELD

The disclosed embodiments relate to a guide wire, and a guide wire manufacturing method.

### BACKGROUND ART

There has been a known guide wire that is used for inserting a catheter or the like into a blood vessel. Such a guide wire requires flexibility and restorability against bending, torquability and pushability for transmitting an operation of the guide wire on a proximal portion to a distal end side, and strong kink resistance against deformation due to bend, wrinkle, and crushing. Incidentally, the torquability and the pushability are also collectively referred to as "operability". For example, Patent Literatures 1 to 3 disclose that a guide wire is equipped with a first core shaft (first wire) disposed on a distal end side, a second core shaft (second wire) disposed on a proximal end side of the first core shaft and joined to the first core shaft, and a covering member (tubular member) for covering a joint part between the first and second core shafts, so that flexibility and operability can be improved.

### CITATION LIST

### Patent Literature

Patent Literature 1: Japanese Patent Laid-Open No. 2004-16359
Patent Literature 2: Japanese Unexamined Patent Application Publication (Translation of PCT Application) No. 2017-513604
Patent Literature 3: U.S. Patent No. 6602208

### SUMMARY OF INVENTION

### Problem to be Solved

However, in the guide wires described in Patent Literatures 1 and 2, a covering member is fixed to first and second core shafts by filling a gap between outer peripheral faces of the first and second core shafts and an inner peripheral face of the covering member with a fixation material (solder agent, solder, adhesive). Thus, a pressure inside the covering member increases due to the excessively filled fixation material, and there has been a possibility for breakage of the covering member. On the other hand, in the guide wire described in Patent Literature 3, holes are formed on the covering member, and therefore the pressure inside the covering member is less likely to increase. However, in the guide wire described in Patent Literature 3, stress concentration is caused around the holes of the covering member e.g. when bending the guide wire, and therefore there has still been the possibility for breakage of the covering member. Incidentally, such problems are not limited to vascular systems, and are common to guide wires to be inserted into each organ in a human body, such as a lymphatic system, a biliary system, a urinary system, a respiratory system, a digestive system, a secretory gland, and a genital organ.

The disclosed embodiments have been made to solve the aforementioned problems, and an object of the disclosed embodiments is to prevent breakage of a covering member in a guide wire including first and second core shafts covered by the covering member.

### Solution to Problem

The disclosed embodiments have been made to solve at least a part of the aforementioned problems, and can be achieved as the following aspects.
(1) An aspect of the disclosed embodiments provides a guide wire. This guide wire includes: a first core shaft disposed on a distal end side and having a first large-diameter portion and a first small-diameter portion with a diameter smaller than of the first large-diameter portion; a second core shaft disposed on a proximal end side and having a second large-diameter portion and a second small-diameter portion with a diameter smaller than of the second large-diameter portion; a covering member that covers a contact portion, where the first small-diameter portion and the second small-diameter portion are arranged oppositely to each other, and at least a part of each of the first small-diameter portion and the second small-diameter portion, the part being adjacent to the contact portion; and a joint layer that joins the covering member with the first small-diameter portion and the second small-diameter portion inside the covering member. A length in an axial direction of the covering member is shorter than a sum of the length in the axial direction of the first small-diameter portion and the length in the axial direction of the second small-diameter portion, and longer than a shorter one of the length in the axial direction of the first small-diameter portion and the length in the axial direction of the second small-diameter portion.
   According to this configuration, the length in the axial direction of the covering member is shorter than the sum of the lengths in the axial direction of the first and second small-diameter portions, and longer than the shorter one of the lengths in the axial direction of the first and second small-diameter portions. Thereby, according to this configuration, when manufacturing the guide wire, in a state that the covering member is introduced to one with a longer length in the axial direction of the first and second small-diameter portions, the first and second small-diameter portions are facing each other, a joining agent is applied onto surfaces of the first and second small-diameter portions, and then the covering member can be moved (slid) to desired positions on the surfaces of the first and second small-diameter portions. As a result, the covering member can easily cover the contact portion between the first and second small-diameter portions by sliding the covering member to an end portion, which is also the large-diameter portion, of the shorter one of the first and second small-diameter portions.
   Furthermore, according to this configuration, the covering member is moved on the surfaces of the first and second small-diameter portions where the joining agent is applied, so that the extra joining agent applied onto the surface is extruded and discharged by an end portion of the covering member. Thus, when the guide wire is manufactured in this way, increase in the pressure inside the covering member due to the extra joining agent (fixation material) charged into the inside of the covering member can be prevented without forming holes responsible for stress concentration on the covering member. As a result, according to this configuration, breakage of the covering member can be prevented in the guide wire including the first and second core shafts covered by the covering member. In addition, this configuration eliminates the step of forming holes on the covering member, so that a number of the manufacturing steps for the guide wire can be reduced.
(2) In the guide wire according to the aforementioned aspect, the first small-diameter portion has a length in the axial direction shorter than the length in the axial direction of the second small-diameter portion, and a bending rigidity of a material for forming the first core shaft may be lower than the bending rigidity of a material for forming the second core shaft. According to this configuration, the shorter of the first and second small-diameter portions is disposed on the distal end side of the guide wire, and further the material constituting the first core shaft on the distal end side has a lower bending rigidity than of the material constituting the second core shaft on the proximal end side. Thereby, flexibility of the guide wire on the distal end side can be increased.
(3) In the guide wire according to the aforementioned aspect, the first core shaft may be made of a superelastic material. According to this configuration, the first core shaft positioned on the distal end side of the guide wire is made of, for example, a superelastic alloy such as a nickel-titanium (NiTi) alloy, therefore the distal end side of the guide wire becomes less susceptible to plastic deformation, and as a result, breakage of the guide wire on the distal end side can be prevented.
(4) In the guide wire according to the aforementioned aspect, the covering member is disposed to position one end of the covering member in a vicinity of a boundary between the first large-diameter portion and the first small-diameter portion, and the joint layer may join at least the contact portion, the first small-diameter portion, and the second small-diameter portion together inside the covering member. According to this configuration, since the joint layer joins the contact portion with the first core shaft (first small-diameter portion) and the second core shaft (second small-diameter portion) inside the covering member, an operation of the guide wire on a proximal portion can be transmitted to the distal end side, and torquability and pushability (operability) can be improved. In addition, even if the first and second core shafts have different rigidities, the covering member can reduce this rigidity gap, so that deformation (bend, wrinkle, crushing) of the first and second core shafts around the contact portion can be prevented, and kink resistance can be improved.
(5) In the guide wire according to the aforementioned aspect, the covering member is disposed in a vicinity of a center between the first small-diameter portion and the second small-diameter portion joined to each other, and the joint layer may join at least both end portions of the covering member with the first small-diameter portion and the second small-diameter portion, respectively, inside the covering member. According to this configuration, since the joint layer joins the both end portions of the covering member with the first core shaft (first small-diameter portion) and the second core shaft (second small-diameter portion) inside the covering member, the operation of the guide wire on the proximal portion can be transmitted to the distal end side, so that torquability and pushability (operability) can be improved. In addition, even when the first and second core shafts have different rigidities, the covering member can reduce this rigidity gap, so that deformation of the first and second core shafts around the contact portion can be prevented, and kink resistance can be improved.
(6) In the guide wire according to the aforementioned aspect, the first small-diameter portion includes a first end portion-side small-diameter portion disposed on an end portion side of the first core shaft, and a first intermediate small-diameter portion disposed between the first end portion-side small-diameter portion and the first large-diameter portion and having a diameter larger than of the first end portion-side small-diameter portion. The second small-diameter portion includes a second end portion-side small-diameter portion disposed on an end portion side of the second core shaft, and a second intermediate small-diameter portion disposed between the second end portion-side small-diameter portion and the second large-diameter portion and having a diameter larger than of the second end portion-side small-diameter portion. Furthermore, an inner covering member that covers the contact portion as well as the first end portion-side small-diameter portion and the second end portion-side small-diameter portion, which are adjacent to the contact portion, may be disposed inside the covering member. According to this configuration, the inner covering member that covers the contact portion and the first and second end portion-side small-diameter portions adjacent to the contact portion is disposed inside the covering member. Thus, even if the first and second core shafts have different rigidities, the inner covering member can reduce the rigidity gap between the first and second core shafts. In addition, even if the first and second core shafts and the covering member have different rigidities, the inner covering member can reduce the rigidity gap thereamong. Thereby, deformation of the first and second core shafts around the contact portion and deformation of the first and second core shafts around the inner covering member can be prevented, so that kink resistance can be further improved.
(7) In the guide wire according to the aforementioned aspect, an outer diameter of the covering member may be substantially equal to a diameter of the first large-diameter portion. According to this configuration, since the outer diameter of the covering member is substantially equal to the diameter of the first large-diameter portion, a surface shape of a connection portion between the first core shaft and the covering member can be formed into a flat shape without unevenness, and when pushing and advancing a medical device such as a balloon catheter through the guide wire, the medical device is prevented from being caught, and as a result, breakage of the medical device can be prevented.
(8) In the guide wire according to the aforementioned aspect, the covering member may be a tubular member having a tubular shape and made of a superelastic material. According to this configuration, the covering member is made of a superelastic material and formed into the tube shape, so that the effect of reducing the rigidity gap can be enhanced, and kink resistance can be further improved.
(9) An aspect of the disclosed embodiments provides a guide wire manufacturing method. This method includes: a preparation step of preparing a first core shaft having a first large-diameter portion and a first small-diameter portion with a diameter smaller than of the first large-diameter portion, a second core shaft having a second large-diameter portion and a second small-diameter portion with a diameter smaller than of the second large-diameter portion, and a covering member having a length in an axial direction that is shorter than a sum of the length in the axial direction of the first small-diameter portion and the length in the axial direction of the second small-diameter portion and longer than a shorter one of the length in the axial direction of the first small-diameter portion and the length in the axial direction of the second small-diameter portion; an introduction step of introducing the covering member from a side of the second small-diameter portion of the second core shaft; an arrangement step of arranging the first small-diameter portion of the first core shaft and the second small-diameter portion of the second core shaft oppositely to each other; an application step of applying a joining agent onto a surface of a contact portion, where the first small-diameter portion and the second small-diameter portion are arranged oppositely to each other, and a surface of each of the first small-diameter portion and the second small-diameter portion, the surface being adjacent to the contact portion and being exposed from the covering member; and a movement step of moving the covering member to positions where the joining agent is applied. According to this method, the guide wire including the first and second core shafts covered by the covering member and capable of preventing breakage of the covering member can be manufactured, and a step of forming holes on the covering member is unnecessary, so that a number of the manufacturing steps for the guide wire can be reduced.
(10) An aspect of the disclosed embodiments provides a guide wire manufacturing method. This method includes: a preparation step of preparing a first core shaft having a first large-diameter portion and a first small-diameter portion with a diameter smaller than of the first large-diameter portion, a second core shaft having a second large-diameter portion and a second small-diameter portion with a diameter smaller than of the second large-diameter portion, and a covering member having a length in an axial direction that is shorter than a sum of the length in the axial direction of the first small-diameter portion and the length in the axial direction of the second small-diameter portion and longer than a shorter one of the length in the axial direction of the first small-diameter portion and the length in the axial direction of the second small-diameter portion; an introduction step of introducing the covering member from a side of the second small-diameter portion of the second core shaft; an arrangement step of arranging the first small-diameter portion of the first core shaft and the second small-diameter portion of the second core shaft oppositely to each other; a first application step of applying a joining agent onto a surface of the first small-diameter portion that is exposed from the covering member; a first movement step of moving the covering member to a position where the joining agent is applied; a second application step of applying the joining agent onto a surface of the second small-diameter portion that is exposed from the covering member; and a second movement step of moving the covering member in a vicinity of a center between the first small-diameter portion and the second small-diameter portion. According to this method, the guide wire including the first and second core shafts covered by the covering member and capable of preventing breakage of the covering member can be manufactured, and a step of forming holes on the covering member is unnecessary, so that a number of the manufacturing steps for the guide wire can be reduced.

Incidentally, the disclosed embodiments can be achieved in various aspects, e.g. in a form of a core shaft product composed of a plurality of core shafts used for a guide wire, a method for manufacturing the core shaft product, or the like.

### BRIEF DESCRIPTION OF DRAWINGS

FIG. 1 is a partial sectional view illustrating an overall configuration of a guide wire according to the first embodiment.
FIG. 2 is a partial sectional view illustrating a vicinity of a contact portion (FIG. 1).
FIGS. 3A, 3B, 3C, and 3D are diagrams for explaining a method for manufacturing the guide wire.
FIG. 4 is a partial sectional view illustrating an overall configuration of a guide wire according to the second embodiment.
FIG. 5 is a partial sectional view illustrating a vicinity of a contact portion according to the second embodiment (FIG. 4).
FIGS. 6A, 6B, 6C, 6D, and 6E are diagrams for explaining a method for manufacturing the guide wire according to the second embodiment.
FIG. 7 is a partial sectional view illustrating an overall configuration of a guide wire according to the third embodiment.
FIG. 8 is a partial sectional view illustrating a vicinity of a contact portion according to the third embodiment (FIG. 7).
FIG. 9 is a partial sectional view illustrating a vicinity of a contact portion in a guide wire according to the fourth embodiment.
FIG. 10 is a perspective view illustrating a schematic configuration of an inner covering member.
FIG. 11 is a partial sectional view illustrating a vicinity of a contact portion in a guide wire according to the fifth embodiment.
FIG. 12 is a partial sectional view illustrating a vicinity of a contact portion in a guide wire according to the sixth embodiment.
FIG. 13 is a partial sectional view illustrating a vicinity of a contact portion in a guide wire according to the seventh embodiment.
FIG. 14 is a partial sectional view illustrating a vicinity of a contact portion in a guide wire according to the eighth embodiment.
FIG. 15 is a partial sectional view illustrating a vicinity of a contact portion in a guide wire according to the ninth embodiment.
FIG. 16 is a partial sectional view illustrating a vicinity of a contact portion in a guide wire according to the tenth embodiment.
FIG. 17 is an enlarged view illustrating a vicinity of a covering member in a guide wire according to the eleventh embodiment.

### EMBODIMENTS OF THE INVENTION

### <First Embodiment>

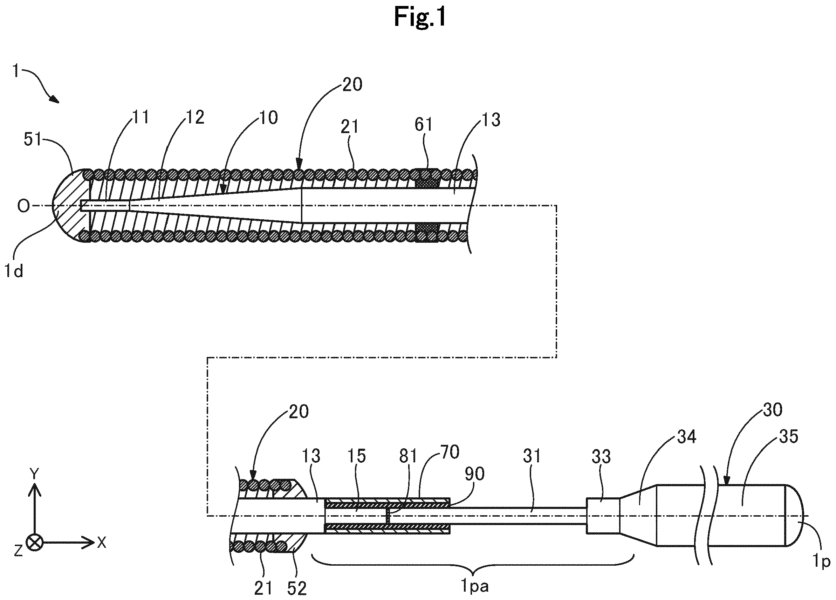

FIG. 1 is a partial sectional view illustrating an overall configuration of a guide wire 1 according to the first embodiment. The guide wire 1 is, for example, a medical appliance used for inserting a catheter into a blood vessel, and includes a first core shaft 10, a coil body 20, a second core shaft 30, a distal end-side fixation portion 51, a proximal end-side fixation portion 52, and a covering member 70. In FIG. 1, an axis passing through a center of the guide wire 1 is represented by an axis line O (dot and dash line). In the following examples, all of an axis passing through a center of each member, the first core shaft 10, the coil body 20, the second core shaft 30, and the covering member 70 coincide with the axis line O. However, the axis passing through the center of each of these members may be inconsistent with the axis line O.

In addition, XYZ axes that are orthogonal to each other are illustrated in FIG. 1. The X axis corresponds to the axial direction of the guide wire 1, the Y axis corresponds to a height direction of the guide wire 1, and the Z axis corresponds to a width direction of the guide wire 1. The left side (-X axis direction) of FIG. 1 is referred to as "distal end side" of the guide wire 1 and each component, and the right side of FIG. 1 (+X axis direction) is referred to as "proximal end side" of the guide wire 1 and each component. In addition, regarding the guide wire 1 and each component, the end portion positioned on the distal end side is referred to as "distal end portion" or simply "distal end", and the end portion positioned on the proximal end side is referred to as "proximal end portion" or simply "proximal end". In the present embodiment, the distal end side corresponds to "farther side", and the proximal end side corresponds to "nearer side". These regards are also common to the figures illustrating the overall configuration in FIG. 1 and the following figures.

The first core shaft 10 is a long member having a large diameter at the center and small diameters on the both end sides (distal end side, proximal end side). The first core shaft 10 is made of a superelastic material e.g. a nickel-titanium (NiTi) alloy, or an alloy of NiTi and another metal. The first core shaft 10 has a distal small-diameter portion 11, a distal decreasing-diameter portion 12, a first large-diameter portion 13, and a first small-diameter portion 15 in this order from the distal end side to the proximal end side. An outer diameter and a length of each portion can be arbitrarily determined.

The distal small-diameter portion 11 is disposed on the distal end portion of the first core shaft 10. The distal small-diameter portion 11 is a part where the outer diameter of the first core shaft 10 is smallest, and has a substantially cylindrical shape with a constant outer diameter. The distal decreasing-diameter portion 12 is disposed between the distal small-diameter portion 11 and the first large-diameter portion 13. The distal decreasing-diameter portion 12 has a substantially truncated cone shape with an outer diameter decreasing from the proximal end side to the distal end side. The first large-diameter portion 13 is disposed between the distal decreasing-diameter portion 12 and the first small-diameter portion 15. The first large-diameter portion 13 is a part where the outer diameter of the first core shaft 10 is largest, and has a substantially cylindrical shape with a constant outer diameter. The first small-diameter portion 15 is disposed on the proximal end portion of the first core shaft 10. The first small-diameter portion 15 has a substantially cylindrical shape with a constant outer diameter smaller than of the first large-diameter portion 13 and larger than of the distal small-diameter portion 11.

The second core shaft 30 is a long tapered member having a large diameter on the proximal end side and a small diameter on the distal end side. The second core shaft 30 is made of a material having a higher rigidity than of the first core shaft 10, e.g. a stainless steel alloy such as SUS304 and SUS316. The second core shaft 30 has a second small-diameter portion 31, a second large-diameter portion 33, a proximal decreasing-diameter portion 34, a proximal large-diameter portion 35 in this order from the distal end side to the proximal end side. An outer diameter and a length of each portion can be arbitrarily determined.

The second small-diameter portion 31 is disposed on the distal end portion of the second core shaft 30. The second small-diameter portion 31 is a part where the outer diameter of the first core shaft 30 is smallest, and has a substantially cylindrical shape with a constant outer diameter substantially equal to that of the first small-diameter portion 15 of the first core shaft 10. The second large-diameter portion 33 is disposed between the second small-diameter portion 31 and the proximal decreasing-diameter portion 34. The second large-diameter portion 33 has a substantially cylindrical shape with a constant outer diameter smaller than of the proximal large-diameter portion 35 and larger than of the second small-diameter portion 31. The proximal decreasing-diameter portion 34 is disposed between the second large-diameter portion 33 and the proximal large-diameter portion 35. The proximal decreasing-diameter portion 34 has a substantially truncated cone shape with an outer diameter decreasing from the proximal end side to the distal end side. The proximal large-diameter portion 35 is disposed on the proximal end portion of the second core shaft 30. The proximal large-diameter portion 35 is a part where the outer diameter of the second core shaft 30 is largest, and has a substantially cylindrical shape with a constant outer diameter.

In the first core shaft 10, the distal small-diameter portion 11, the distal decreasing-diameter portion 12, and the distal end side of the first large-diameter portion 13 are covered by the coil body 20 described later. On the other hand, the proximal end side of the first large-diameter portion 13 and the first small-diameter portion 15 in the first core shaft 10, and each portion in the second core shaft 30 are not covered by the coil body 20 but are exposed from the coil body 20. The proximal large-diameter portion 35 in the second core shaft 30 is used when an operator grasps the guide wire 1.

The coil body 20 has a substantially hollow cylindrical shape formed by spirally winding a wire 21 around the first core shaft 10. The wire 21 constituting the coil body 20 may be a solid wire composed of one wire, or a twisted wire obtained by twisting a plurality of wires. When the wire 21 is a solid wire, the coil body 20 is configured as a single coil, and when the wire 21 is a twisted wire, the coil body 20 is configured as a hollow twisted wire coil. Alternatively, the coil body 20 may be configured by combining the single coil and the hollow twisted wire coil. The wire diameter of the wire 21 and an average coil diameter of the coil body 20 (average diameter of the outer diameter and the inner diameter of the coil body 20) can be arbitrarily determined.

The wire 21 can be made of, for example, a stainless steel alloy such as SUS304 and SUS316, a superelastic alloy such as an NiTi alloy, a piano wire, a radiolucent alloy such as nickel-chromium alloy and cobalt alloy, gold, platinum, tungsten, or a radiopaque alloy such as an alloy including the aforementioned elements (e.g. platinum-nickel alloy). Incidentally, the wire 21 may be made of a known material other than the aforementioned materials.

The distal end-side fixation portion 51 is disposed on the distal end portion of the guide wire 1 and integrally holds the distal end portion of the distal small-diameter portion 11 of the first core shaft 10, and the distal end portion of the coil body 20. The distal end-side fixation portion 51 can be formed from any joining agent, e.g. a metal solder such as silver solder, gold solder, zinc, Sn-Ag alloy, and Au-Sn alloy, or an adhesive such as epoxy adhesive. The proximal end-side fixation portion 52 is disposed on a part closer to the proximal end side of the first large-diameter portion 13 of the first core shaft 10 and integrally holds the first core shaft 10 and the proximal end portion of the coil body 20. The proximal end-side fixation portion 52 can be formed from any joining agent in the same manner as for the distal end-side fixation portion 51. For the proximal end-side fixation portion 52 and the distal end-side fixation portion 51, the same joining agent or different joining agents may be used.

The intermediate fixation portion 61 integrally holds the coil body 20 and the first core shaft 10 in the vicinity of the intermediate portion of the coil body 20 in the axis line O direction. The intermediate fixation portion 61 can be formed from any joining agent in the same manner as for the distal end-side fixation portion 51. For the intermediate fixation portion 61 and the distal end-side fixation portion 51, the same joining agent or different joining agents may be used. Although one intermediate fixation portion 61 has been described as an example in FIG. 1, a plurality of intermediate fixation portions 61 may be disposed on the guide wire 1.

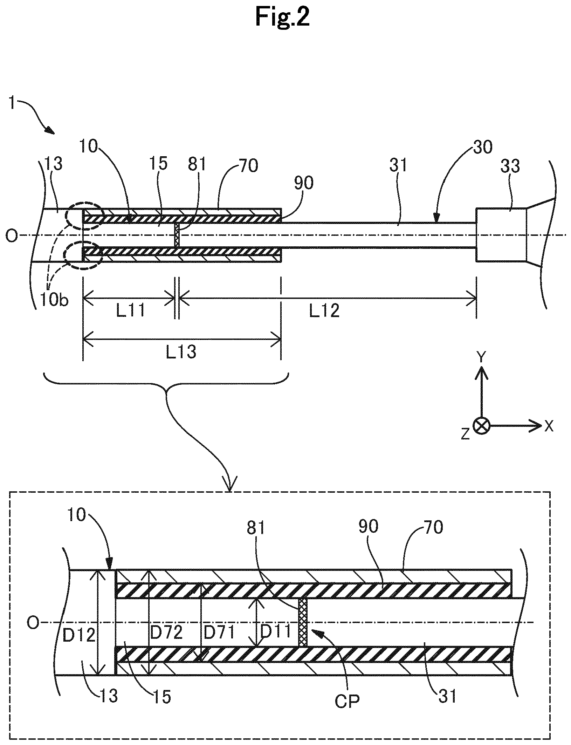

FIG. 2 is a partial sectional view illustrating a vicinity 1pa of the contact portion (FIG. 1). In FIG. 2, a partial sectional view illustrating the vicinity of the contact portion between the first and second core shafts 10 and 30 is illustrated in an upper column, and an enlarged view of the vicinity of the covering member 70 is illustrated in a lower column. The XYZ axes illustrated in FIG. 2 correspond to the XYZ axes respectively in FIG. 1. The same applies to the figures with XYZ axes in FIG. 2 and the following figures.

The first and second core shafts 10 and 30 are facing each other (counter arrangement) such that their central axes coincide with each other. In the example of the figure, each central axis of the first and second core shafts 10 and 30 coincides with the axis line O. However, the central axes of the first and second core shafts 10 and 30 may coincide with each other at a position on a YZ plane different from the axis line O. In addition, the central axes of the first and second core shafts 10 and 30 may be inconsistent with each other. Hereinafter, a part where the first and second core shafts 10 and 30 are facing each other will be referred to as "contact portion CP". In the example of the figure, the contact portion CP is a part where the proximal end portion of the first small-diameter portion 15 in the first core shaft 10 and the distal end portion of the second small-diameter portion 31 in the second core shaft 30 are adjacent to each other.

In the present embodiment, the first and second core shafts 10 and 30 are joined to each other on the contact portion CP. The joining can be performed, for example, in such a way that a gap between the first small-diameter portion 15 of the first core shaft 10 and the second small-diameter portion 31 of the second core shaft 30 adjacent to each other on the contact portion CP is filled with a joining agent 81 and the joining agent 81 is hardened. In this case, the joining agent 81 may be applied on the whole gap between the first small-diameter portion 15 and the second small-diameter portion 31 (in other words, the whole face between the end face on the proximal end side of the first small-diameter portion 15 and the end face on the distal end side of the second small-diameter portion 31), and alternatively the joining agent 81 may be applied only on a part of the gap and the other part may be a void. As the joining agent 81, for example, a metal solder such as silver solder, gold solder, zinc, Sn-Ag alloy, and Au-Sn alloy, or an adhesive such as epoxy adhesive can be used. In addition, joining on the contact portion CP may be performed by welding the first and second core shafts 10 and 30.

Incidentally, the first and second core shafts 10 and 30 are not necessarily joined to each other on the contact portion CP. In this case, on the contact portion CP, the end face on the proximal end side of the first small-diameter portion 15 and the end face on the distal end side of the second small-diameter portion 31 may be in contact with each other, or alternatively the end face on the proximal end side of the first small-diameter portion 15 and the end face on the distal end side of the second small-diameter portion 31 may be adjacent to each other via a void therebetween.

The covering member 70 is a metal tubular member formed into a tube shape (substantially hollow cylindrical shape) having a constant outer diameter. The covering member 70 is preferably configured so as to have a lower bending rigidity than of the second core shaft 30, and have an elastic modulus equivalent to that of the first core shaft 10. Similarly to the first core shaft 10, the covering member 70 can be made of a superelastic material, e.g. an NiTi alloy, or an alloy of NiTi and another metal. The covering member 70 may be made of a material that is the same as or different from that of the first core shaft 10.

The covering member 70 is disposed such that one end (in the example of the figure, distal end portion) of the covering member 70 is positioned in a vicinity 10b of a boundary between the first large-diameter portion 13 and the first small-diameter portion 15 in the first core shaft 10. In addition, the covering member 70 covers the contact portion CP between the first and second core shafts 10 and 30, and a part of the first core shaft 10 adjacent to the contact portion CP (in the example of the figure, the distal end side of the proximal end portion of the first small-diameter portion 15), and a part of the second core shaft 30 (in the example of the figure, a part on the proximal end side of the distal end portion of the second small-diameter portion 31). In other words, the first and second core shafts 10 and 30 joined to each other on the contact portion CP extend in the axis line O direction so as to pass through an inner cavity of the covering member 70.

The joint layer 90 is disposed inside the covering member 70, and joins and integrally holds the covering member 70 and the first and second core shafts 10 and 30 (first small-diameter portion 15 and second small-diameter portion 31). In the example of the figure, the joint layer 90 is formed on the whole gap between the covering member 70 and the first and second core shafts 10 and 30 covered by the covering member 70, and joins the contact portion CP between the first and second small-diameter portions 15 and 31, the first small-diameter portion 15 of the first core shaft 10 adjacent to the contact portion CP, and the second small-diameter portion 31 of the second core shaft 30 adjacent to the contact portion CP. Incidentally, the joint layer 90 may be formed on not the whole but a part of the gap between the covering member 70 and the first and second core shafts 10 and 30. When the joint layer 90 is formed on a part, a void may remain between the covering member 70 and the first and second core shafts 10 and 30. The joint layer 90 can be formed from any joining agent similarly to the distal end-side fixation portion 51. For the joint layer 90 and the distal end-side fixation portion 51, the same joining agent or different joining agents may be used.

As illustrated in the upper column of FIG. 2, in the first embodiment, a length L11 in the axis line O direction of the first small-diameter portion 15 in the first core shaft 10 is shorter than a length L12 of the second small-diameter portion 31 in the axis line O direction in the second core shaft 30 (L11<L12). In addition, a length L13 of the covering member 70 in the axis line O direction is shorter than a sum of the length L11 of the first small-diameter portion 15 in the axis line O direction and the length L12 of the second small-diameter portion 31 axis line O direction (L13<L11+L12). Furthermore, the length L13 of the covering member 70 in the axis line O direction is longer than a shorter one of the lengths (i.e. the length L11 of the first small-diameter portion 15) in the axis line O direction of the first and second small-diameter portions 15 and 31 (L13>L11). Incidentally, the lengths L11, L12, and L13 can be arbitrarily determined as long as they satisfy the aforementioned relationships.

As illustrated in the lower column of FIG. 2, in the first embodiment, an inner diameter D71 of the covering member 70 is larger than a diameter D11 of the first small-diameter portion 15 in the first core shaft 10 (D71>D11). In addition, an outer diameter D72 of the covering member 70 is substantially equal to a diameter D12 of the first large-diameter portion 13 in the first core shaft 10 (D72=D12). Incidentally, the inner diameter D71, the outer diameter D72, and the diameters D11 and D12 can be arbitrarily determined as long as they satisfy the aforementioned relationships.

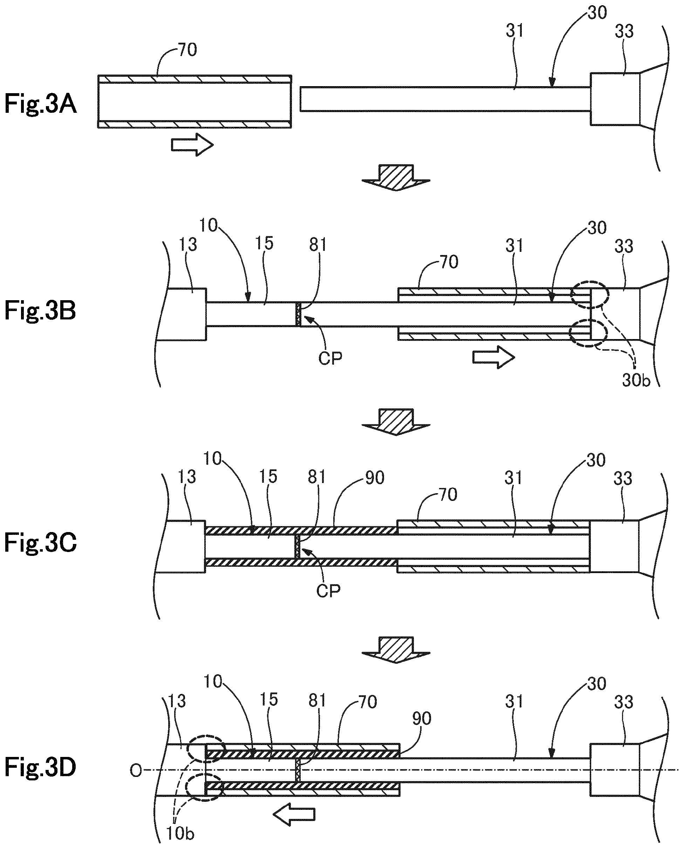

FIGS. 3A, 3B, 3C, and 3D are diagrams for explaining a method for manufacturing the guide wire 1. FIG. 3A illustrates an introduction step. Similarly, FIG. 3B illustrates a disposition step, FIG. 3C illustrates an application step, and FIG. 3D illustrates a movement step. First, in a preparation step, the first core shaft 10 having the first large-diameter portion 13 and the first small-diameter portion 15, the second core shaft 30 having the second large-diameter portion 33 and the second small-diameter portion 31, and the tubular covering member 70 having the aforementioned length L13 are previously prepared.

Next, as indicated by the white arrow in FIG. 3A, the covering member 70 is introduced from the side of the second small-diameter portion 31 in the second core shaft 30 (introduction step). In the example of FIG. 3B, the covering member 70 is moved (slid) until the proximal end portion of the covering member 70 is positioned in a vicinity 30b of a boundary between the second small-diameter portion 31 and the second large-diameter portion 33 in the second core shaft 30. Subsequently, as illustrated in FIG. 3B, the first small-diameter portion 15 of the first core shaft 10 is disposed so as to be facing the second small-diameter portion 31 to which the covering member 70 is introduced in the second core shaft 30 (disposition step). Then, the joining agent 81 is applied onto the part (contact portion CP) where the first small-diameter portion 15 and the second small-diameter portion 31 are facing each other, to join the first and second core shafts 10 and 30.

Subsequently, as illustrated in FIG. 3C, the joining agent for forming the joint layer 90 is applied onto the surface of the contact portion CP and each surface of the first and second small-diameter portions 15 and 31 adjacent to the contact portion CP and exposed from the covering member 70 (in the example of the figure, the surface of the first small-diameter portion 15, and the surface on the distal end side of the second small-diameter portion 31 (application step). In the example of the figure, the joining agent is applied onto the whole surfaces of the first and second small-diameter portions 15 and 31 exposed from the covering member 70, but the joining agent may be applied onto a part of the surfaces of the first and second small-diameter portions 15 and 31. Subsequently, as indicated by the white arrow in FIG. 3D, the covering member 70 is moved (slid) to the position where the joining agent for forming the joint layer 90 is applied (Movement step). In the example of the figure, the covering member 70 is moved until the distal end portion of the covering member 70 is positioned in the vicinity 10b of the boundary between the first large-diameter portion 13 and the first small-diameter portion 15 in the first core shaft 10.

As described above, in the guide wire 1 according to the first embodiment, the length L13 in the axis line O direction of the covering member 70 is shorter than the sum of the lengths L11 and L12 of the first and second small-diameter portions 15 and 31 in the axis line O direction, longer than the shorter of the lengths of the first and second small-diameter portions 15 and 31 in the axis line O direction (in the example of the figure, the length L11 of the first small-diameter portion 15), and shorter than the longer of the lengths in the axis line O direction (in the example of the figure, the length L12 of the second small-diameter portion 31) (upper column of FIG. 2). Thus, as explained in FIGS. 3A, 3B, 3C, and 3D, when manufacturing the guide wire 1 according to the first embodiment, in a state that the covering member 70 is introduced to the one of the first and second small-diameter portions 15 and 31 with the longer length in the axis line O direction (in the aforementioned example, the second small-diameter portion 31) (FIG. 3A), the first and second small-diameter portions 15 and 31 are facing each other (FIG. 3B), the joining agent is applied onto the surfaces of the first and second small-diameter portions 15 and 31 (FIG. 3C), and then the covering member 70 can be moved to desired positions on the surfaces of the first and second small-diameter portions 15 and 31 (FIG. 3D). As a result, the covering member 70 can easily cover the contact portion CP between the first and second small-diameter portions 15 and 31 by sliding the covering member 70 to an end portion, which is also the large-diameter portion, of the shorter one of the first and second small-diameter portions 15 and 31 (in the aforementioned example, the first small-diameter portion 15) (in other words, the opposite end portion to the contact portion CP).

As illustrated in FIG. 3D, the covering member 70 is moved on the surfaces of the first and second small-diameter portions 15 and 31 where the joining agent is applied, so that the extra joining agent applied onto the surfaces of the first and second small-diameter portions 15 and 31 is extruded and discharged by an end portion (end face) of the covering member 70. Thus, when the guide wire 1 is manufactured as illustrated in FIGS. 3A, 3B, 3C, and 3D, increase in the pressure inside the covering member 70 due to the extra joining agent (fixation material) charged into the inside of the covering member 70 can be prevented without forming holes responsible for a stress concentration on the covering member 70. As a result, according to the first embodiment, breakage of the covering member 70 can be prevented in the guide wire 1 including the first and second core shafts 10 and 30 covered by the covering member 70. In addition, according to the present embodiment, the step of forming holes on the covering member 70 is unnecessary, so that a number of the manufacturing steps for the guide wire 1 can be reduced.

Additionally, in the guide wire 1 according to the first embodiment, the shorter one of the first and second small-diameter portions 15 and 31 (in the aforementioned example, the first small-diameter portion 15) is disposed on the distal end side of the guide wire 1, and furthermore, the first core shaft 10 on the distal end side is made of a material having a lower bending rigidity than of a material constituting the second core shaft 30 on the proximal end side. Thereby, flexibility of the guide wire 1 on the distal end side can be increased. In addition, since the first core shaft 10 positioned on the distal end side of the guide wire 1 is made of, for example, a superelastic alloy such as a NiTi alloy, the distal end side of the guide wire 1 becomes less susceptible to plastic deformation, and as a result, breakage of the distal end side of the guide wire 1 can be prevented.

Furthermore, in the guide wire 1 according to the first embodiment, the length L13 of the covering member 70 in the axis line O direction is longer than the length L11 of the first small-diameter portion 15 in the axis line O direction (the upper column of FIG. 2). Thus, when manufacturing the guide wire 1, the joining agent is applied onto the surfaces of the first and second small-diameter portions 15 and 31, then, as illustrated in FIG. 3D, the covering member 70 is moved to the opposite end portion to the contact portion CP of the first small-diameter portion 15 (i.e. distal end portion), so that a configuration that the contact portion CP and the vicinity thereof are covered by the covering member 70 can be easily obtained.

Furthermore, in the guide wire 1 according to the first embodiment, the joint layer 90 joins the contact portion CP, the first core shaft 10 (first small-diameter portion 15), and the second core shaft 30 (second small-diameter portion 31) inside the covering member 70 (upper column of FIG. 2). Thereby, an operation such as turning and pushing of the guide wire 1 on a proximal portion (FIG. 1: proximal large-diameter portion 35) can be transmitted to the distal end side of the guide wire 1, so that torquability and pushability (operability) of the guide wire 1 can be improved. In addition, as in the aforementioned embodiment, even if the first and second core shafts 10 and 30 have different rigidities due to difference in the material between the first and second core shafts 10 and 30, the rigidity gap between the first and second core shafts 10 and 30 can be reduced by the covering member 70. Thereby, deformation (bend, wrinkle, crushing) of the first and second core shafts 10 and 30 around the contact portion CP can be prevented, so that kink resistance of the guide wire 1 can be improved.

Furthermore, in the guide wire 1 according to the present embodiment, the covering member 70 is made of a superelastic material and formed into a tube shape, so that the effect of reducing the rigidity gap by the covering member 70 can be enhanced, and the kink resistance of the guide wire 1 can be further improved. In addition, the first core shaft 10 is made or a superelastic material, so that flexibility and restorability against bending can be improved. The second core shaft 30 is made of a material having a higher rigidity than of the first core shaft 10, so that torquability and the pushability (operability) can be improved.

Furthermore, in the guide wire 1 according to the first embodiment, the outer diameter D72 of the covering member 70 is substantially equal to the diameter D12 of the first large-diameter portion 13 in the first core shaft 10 (lower column of FIG. 2). Thereby, a surface shape of a connection portion between the first core shaft 10 and the covering member 70 can be formed into a flat shape without unevenness, and when pushing and advancing a medical device such as a balloon catheter through the guide wire 1, the medical device is prevented from being caught, and as a result, breakage of the medical device can be prevented.

### <Second Embodiment>

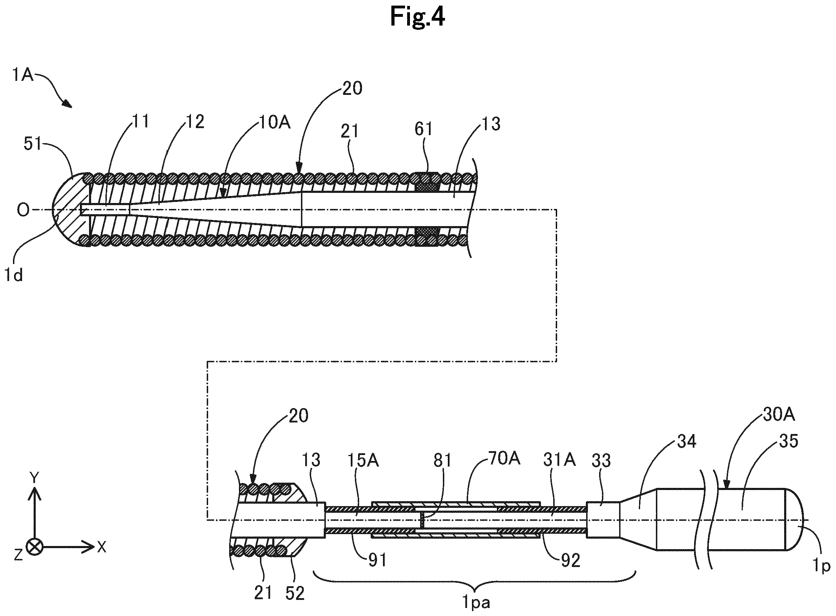

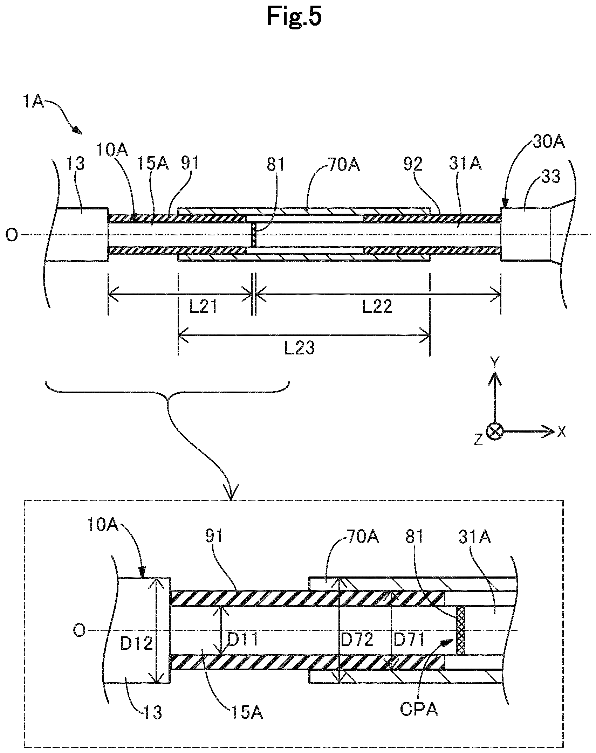

FIG. 4 is a partial sectional view illustrating an overall configuration of a guide wire 1A according to the second embodiment. FIG. 5 is a partial sectional view illustrating a vicinity 1pa of a contact portion according to the second embodiment (FIG. 4). The configurations of the upper and lower columns in FIG. 5 are the same as in FIG. 2. In the guide wire 1A according to the second embodiment, mainly an arrangement of a covering member 70A in the axis line O direction is different from that in the first embodiment.

The covering member 70A is disposed in a vicinity of a center between a first small-diameter portion 15A of a first core shaft 10A and a second small-diameter portion 31A of a second core shaft 30A joined to each other on a contact portion CPA. In other words, a distal end portion of the covering member 70A is disposed at a distance in the axis line O direction from a vicinity of a boundary between a first large-diameter portion 13 and a first small-diameter portion 15A of the first core shaft 10A. In addition, a proximal end portion of the covering member 70A is disposed at a distance in the axis line O direction from a vicinity of a boundary between a second large-diameter portion 33 and a second small-diameter portion 31A of the second core shaft 30A. As in the first embodiment, the covering member 70A covers the contact portion CPA between the first and second core shafts 10A and 30A, a part of the first core shaft 10A adjacent to the contact portion CPA (in the example of the figure, a part on a distal end side of a proximal end portion of the first small-diameter portion 15A), and a part of the second core shaft 30A (in the example of the figure, a part on a proximal end side of a distal end portion of the second small-diameter portion 31A).

In addition, the guide wire 1A includes two joint layers 91 and 92 instead of the joint layer 90 according to the first embodiment. The joint layer 91 joins a part on the distal end side of the covering member 70A with the first core shaft 10A (first small-diameter portion 15A) inside the covering member 70A. The joint layer 92 joins a part on the proximal end side of the covering member 70A with the second core shaft 30A (second small-diameter portion 31A) inside the covering member 70A. In the example of the figure, the joint layer 91 and the joint layer 92 are formed only on both end portions of the covering member 70A in the axis line O direction, and are not formed around the middle part of the covering member 70A. That means, around the middle part of the covering member 70A, there is a void between an inner face of the covering member 70A and outer faces of the first and second small-diameter portions 15A and 31A adjacent to the contact portion CPA. The joint layer 91 and the joint layer 92 can be formed from any joining agent in the same manner as for the distal end-side fixation portion 51. For the joint layers 91 and 92 and the distal end-side fixation portion 51, the same joining agent or different joining agents may be used.

As illustrated in the upper column of FIG. 5, also in the second embodiment, a length L21 of the first small-diameter portion 15A in the axis line O direction in the first core shaft 10A is shorter than a length L22 of the second small-diameter portion 31A in the axis line O direction in the second core shaft 30A (L21<L22), as in the first embodiment. In addition, a length L23 of the covering member 70A in the axis line O direction is shorter than a sum of the length L21 of the first small-diameter portion 15A in the axis line O direction and the length L22 of the second small-diameter portion 31A in the axis line O direction (L23<L21+L22). Furthermore, the length L23 of the covering member 70A in the axis line O direction is longer than the shorter of the lengths of the first and second small-diameter portions 15A and 31A (the length L21 of the first small-diameter portion 15A) in the axis line O direction (L23>L21). The lengths L21, L22, and L23 can be arbitrarily determined as long as they satisfy the aforementioned relationships. Also, a dimensional relationship of an inner diameter D71 and an outer diameter D72 of the covering member 70, a diameter D11 of the first small-diameter portion 15A, and a diameter D12 of the first large-diameter portion 13 is the same as in the first embodiment.

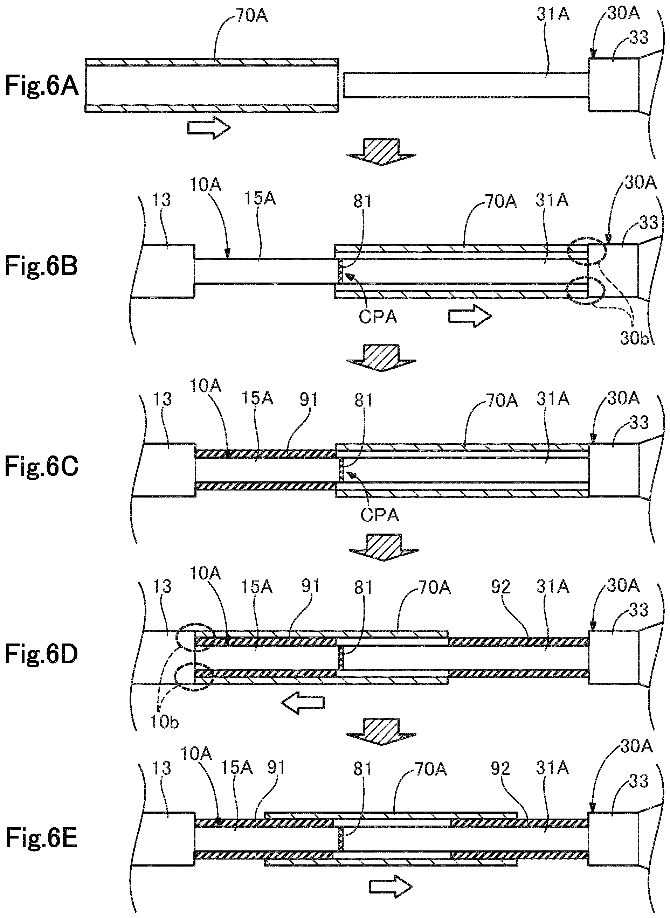

FIGS. 6A, 6B, 6C, 6D, and 6E are diagrams for explaining a method for manufacturing the guide wire 1A according to the second embodiment. FIG. 6A illustrates an introduction step. Similarly, FIG. 6B illustrates a disposition step, FIG. 6C illustrates a first application step, FIG. 6D illustrates a first movement step and a second application step, and FIG. 6E illustrates the second movement step. A preparation step, the introduction step, the disposition step are the same as in the first embodiment explained in FIGS. 3A, 3B, 3C, and 3D.

As illustrated in FIG. 6C, after the disposition step is completed, a joining agent for forming the joint layer 91 is applied onto a surface of the first small-diameter portion 15A exposed from the covering member 70A (first application step). In the example of the figure, although the joining agent is applied onto the whole surface of the first small-diameter portion 15A exposed from the covering member 70A, the joining agent may be applied onto a part of the surface of the first small-diameter portion 15A. Subsequently, as indicated by the white arrow in FIG. 6D, the covering member 70A is moved (slid) to a position where the joining agent for forming the joint layer 91 is applied (first movement step). In the example of the figure, the covering member 70A is moved until the distal end portion of the covering member 70A is positioned in a vicinity 10b of a boundary between the first large-diameter portion 13 and the first small-diameter portion 15A.

As illustrated in FIG. 6D, a joining agent for forming the joint layer 92 is applied onto a surface of the second small-diameter portion 31A exposed from the moved covering member 70A (second application step). In the example of the figure, although the joining agent is applied onto the whole surface of the second small-diameter portion 31A exposed from the covering member 70A, the joining agent may be applied onto a part of the surface of the second small-diameter portion 31A. Finally, as indicated by the white arrow in FIG. 6E, the covering member 70A is moved to a vicinity of a center between the first small-diameter portion 15A and the second small-diameter portion 31A (second movement step).

In this way, the covering member 70A may be disposed at a distance in the axis line O direction from the vicinity of the boundary between the large-diameter portion and the small-diameter portion of the first and second core shafts 10A and 30A. The covering member 70A is not necessarily disposed in the vicinity of the center between the first and second small-diameter portions 15A and 31A. In addition, the covering member 70A may be joined to the first and second core shafts 10A and 30A such that a void is formed between the inner face of the covering member 70A and the outer faces (surfaces) of the first and second core shafts 10A and 30A.

The same effect as in the first embodiment can also be exhibited by this guide wire 1A according to the second embodiment. That means, in the guide wire 1A including the first and second core shafts 10A and 30A covered by the covering member 70A, breakage of the covering member 70A can be prevented, and the step of forming holes on the covering member 70A is unnecessary, so that a number of the manufacturing steps for the guide wire 1A can be reduced. Also, in the guide wire 1A according to the second embodiment, two joint layers 91 and 92 join the both end portions of the covering member 70A and the first and second small-diameter portions 15A and 31A inside the covering member 70A, and therefore an operation of a guide wire 1A on a proximal portion can be transmitted to the distal end side, so that torquability and pushability (operability) of the guide wire 1A can be improved. In addition, even if the first and second core shafts 10A and 30A have different rigidities, this rigidity gap can be reduced by the covering member 70A, and therefore deformation of the first and second core shafts 10A and 30A around the contact portion CPA can be prevented, so that kink resistance of the guide wire 1A can be improved.

### <Third Embodiment>

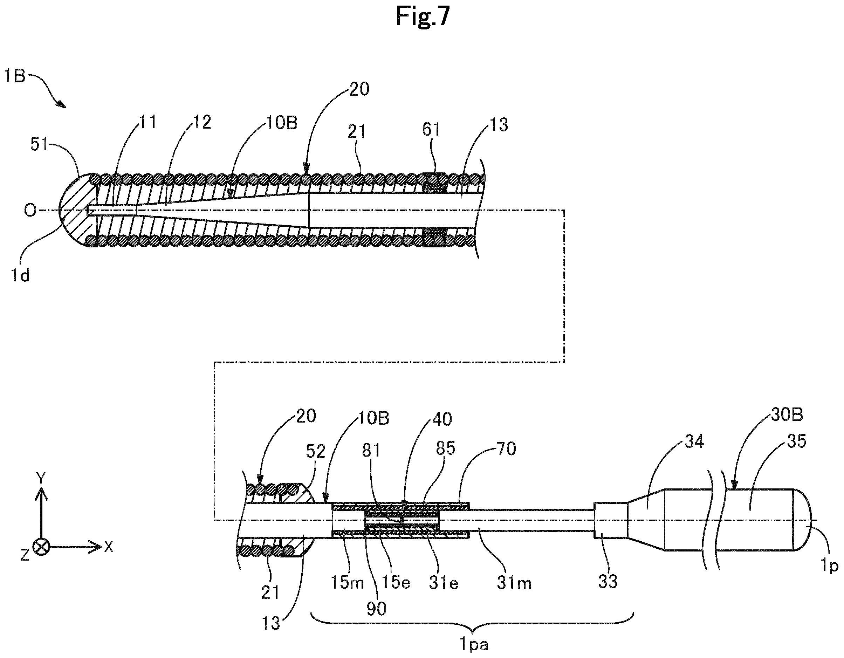

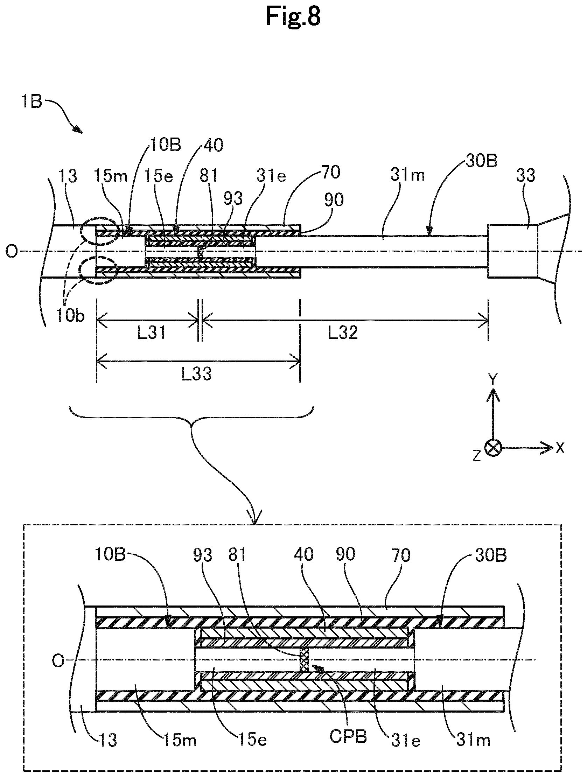

FIG. 7 is a partial sectional view illustrating an overall configuration of a guide wire 1B according to the third embodiment. FIG. 8 is a partial sectional view illustrating the vicinity 1pa of the contact portion according to the third embodiment (FIG. 7). Configurations of the upper and lower columns in FIG. 8 are the same as in FIG. 2. In the guide wire 1B according to the third embodiment, configurations of first and second core shafts 10B and 30B in the vicinity 1pa of the contact portion are different from those in the first embodiment, and the guide wire 1B further includes an inner covering member 40 inside the covering member 70.

In the first core shaft 10B, the first small-diameter portion 15 includes a first end portion-side small-diameter portion 15e and a first intermediate small-diameter portion 15m. The first end portion-side small-diameter portion 15e is disposed on a proximal end portion side of the first core shaft 10B and has a substantially cylindrical shape with a constant outer diameter smaller than of the first intermediate small-diameter portion 15m. The first intermediate small-diameter portion 15m is disposed between the first end portion-side small-diameter portion 15e and the first large-diameter portion 13 and has a substantially cylindrical shape with a constant outer diameter larger than of the first end portion-side small-diameter portion 15e and smaller than of the first large-diameter portion 13. In the second core shaft 30B, the second small-diameter portion 31 includes a second end portion-side small-diameter portion 31e and a second intermediate small-diameter portion 31m. The second end portion-side small-diameter portion 31e is disposed on a distal end portion side of the second core shaft 30B and has a substantially cylindrical shape with a constant outer diameter smaller than of the second intermediate small-diameter portion 31m. The second intermediate small-diameter portion 31m is disposed between the second end portion-side small-diameter portion 31e and the second large-diameter portion 33 and has a substantially cylindrical shape with a constant outer diameter larger than of the second end portion-side small-diameter portion 31e and smaller than of the second large-diameter portion 33.

As illustrated in the lower column of FIG. 8, on a contact portion CPB, an end face on the proximal end side of the first end portion-side small-diameter portion 15e in the first core shaft 10B and an end face on the distal end side of the second end portion-side small-diameter portion 31e in the second core shaft 30B are adjacent to each other. On the contact portion CPB, the first and second core shafts 10B and 30B are joined to each other. For this joining, various aspects can be adopted, or joining may be omitted, in the same manner as in the first embodiment.

The inner covering member 40 is a metal tubular member formed into a tube shape (substantially hollow cylindrical shape) having a constant outer diameter. The inner covering member 40 according to the third embodiment is configured so as to have a lower bending rigidity than of the second core shaft 30B, and have an elastic modulus equivalent to that of the first core shaft 10. Similarly to the covering member 70, the inner covering member 40 can be made of a superelastic material, e.g. an NiTi alloy, or an alloy of NiTi and another metal. The inner covering member 40 may be made of a material that is the same as or different from that of the covering member 70.

The inner covering member 40 is disposed so as to cover the contact portion CPB, a part of the first core shaft 10B adjacent to the contact portion CPB (in the example of the figure, the first end portion-side small-diameter portion 15e), and a part of the second core shaft 30B adjacent to the contact portion CPB (in the example of the figure, the second end portion-side small-diameter portion 31e), inside the covering member 70. In the third embodiment, a length of an inner covering member 40B in the axis line O direction is substantially equal to a sum of a length of the first end portion-side small-diameter portion 15e in the axis line O direction and a length of the second end portion-side small-diameter portion 31e in the axis line O direction. Incidentally, a length of an inner covering member 40D can be arbitrarily determined.

The inner covering member 40 is joined with the first and second core shafts 10B and 30B (first end portion-side small-diameter portion 15e and second end portion-side small-diameter portion 31e) via a joint layer 93 disposed inside the inner covering member 40. In the example of the figure, the joint layer 93 is formed on almost a whole gap between the inner covering member 40 and the first and second end portion-side small-diameter portions 15e and 31e covered by the inner covering member 40 excluding a distal end portion of the first end-side small-diameter portion 15e and a proximal end portion of the second end portion-side small-diameter portion 31e. This joint layer 93 joins the contact portion CPB between the first and second end-side small-diameter portions 15e and 31e with the first and second end-side small-diameter portions 15e and 31e adjacent to the contact portion CPB. The joint layer 93 can be formed from any joining agent similarly to the distal end-side fixation portion 51. For the joint layer 93 and the distal end-side fixation portion 51, the same joining agent or different joining agents may be used.

As illustrated in the upper column of FIG. 8, also in the third embodiment, a length L31 of the first small-diameter portion 15 in the axis line O direction in the first core shaft 10B (i.e. first end portion-side small-diameter portion 15e and first intermediate small-diameter portion 15m) is shorter than a length L32 of the second small-diameter portion 31 in the axis line O direction in the second core shaft 30B (i.e. second end portion-side small-diameter portion 31e and second intermediate small-diameter portion 31m) (L31<L32) as in the first embodiment. Also, the relationship between a length L33 of the covering member 70 in the axis line O direction and the lengths L31 and L32 is the same as in the first embodiment (L33<L31+L32, L33>L31). The lengths L31, L32, and L33 can be arbitrarily determined as long as they satisfy the aforementioned relationships.

In this way, the inner covering member 40 may be further disposed inside the covering member 70. In addition, the first small-diameter portion 15 of the first core shaft 10B and the second small-diameter portion 31 of the second core shaft 30B may be composed of two or more small-diameter portions. The guide wire 1B according to the third embodiment can be manufactured by the same manner as in the first embodiment explained in FIGS. 3A, 3B, 3C, and 3D.

The same effect as in the first embodiment can also be exhibited by this guide wire 1B according to the third embodiment. Furthermore, in the guide wire 1B according to the third embodiment, the inner covering member 40 covering the contact portion CPB and the first and second end portion-side small-diameter portions 15e and 31e is disposed inside the covering member 70. Thereby, even if the first and second core shafts 10B and 30B have different rigidities, the rigidity gap between the first and second core shafts 10B and 30B can be reduced by the inner covering member 40. In addition, even if the first core shaft 10B, the second core shaft 30B, and the covering member 70 have different rigidities, the rigidity gap thereamong can be reduced by the inner covering member 40. Thereby, deformation of the first and second core shafts 10B and 30B around the contact portion CPB and deformation of the first and second core shafts 10B and 30B around the inner covering member 40 can be prevented, so that kink resistance of the guide wire 1B can be further improved.

Furthermore, in a case that the long guide wire 1B is turned around the axis line O on the proximal end side, if each central axis of the covering member 70 and the first and second core shafts 10B and 30B after joining is deviated from the axis line O due to a machining error, the turning of the first core shaft 10B is disordered by grasping the second core shaft 30B of the guide wire 1B and turning the second core shaft 30B around the axis line O as a turning axis. It is desirable that assembly is performed such that such a turning disorder is minimized, but it takes time for assembly.

In the guide wire according to the third embodiment, the outer diameter of the inner covering member 40 and outer diameters of the first and second core shafts 10B and 30B are conformed to each other, so that it is possible to prevent an influence to the turning disorder depending on a machining accuracy of the first end portion-side small-diameter portion 15e of the first core shaft 10B and the second end portion-side small-diameter portion 31e of the second core shaft 30B. In addition, the outer diameter of the inner covering member 40 and the first and second core shafts 10B and 30B are integrally configured, so that it is possible to prevent an influence to the turning disorder depending on an inner face machining accuracy of the covering member 70. Thus, even if machining errors are caused on the inner face of the covering member 70, and outer faces of the first and second end portion-side small-diameter portions 15e and 31e of the first and second core shafts 10B and 30B, the turning disorder of the first core shaft 10B of the guide wire 1B can be prevented.

### <Fourth Embodiment>

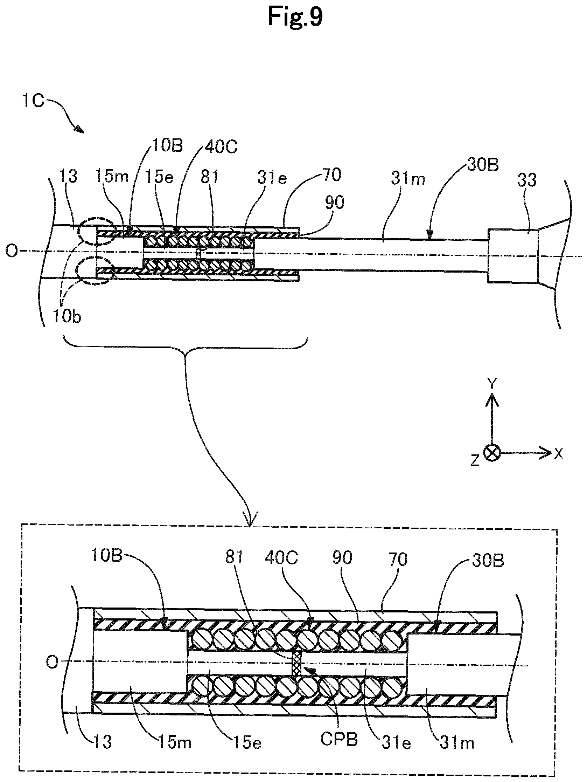

FIG. 9 is a partial sectional view illustrating a vicinity of a contact portion in a guide wire 1C according to the fourth embodiment. The configurations in the upper and lower columns of FIG. 9 are the same as in FIG. 2. The guide wire 1C according to the fourth embodiment includes an inner covering member 40C having a different configuration from that in the third embodiment in the configuration according to the third embodiment.

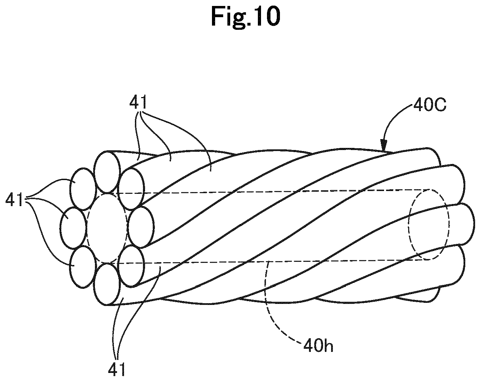

FIG. 10 is a perspective view illustrating a schematic configuration of an inner covering member 40C. The inner covering member 40C is a multi-thread coil obtained by winding eight wires 41, and has a substantially hollow cylindrical shape with a constant outer diameter. The inner covering member 40C is preferably configured to have a lower bending rigidity than of the second core shaft 30B. The inner covering member 40C can be formed, for example. in such a way that the eight wires 41 are tightly twisted around a cored bar so as to be in contact with each other, then a residual stress is removed using a known heat treatment method, and the cored bar is drawn out. The inner covering member 40C formed in this way is a multi-thread coil having an inner cavity 40h (FIG. 10: dashed line) as illustrated in FIG. 10. A material of the wire 41 may be made of a material that is the same as or different from that of the wire 21. Incidentally, for the inner covering member 40C, any aspect can be adopted. For example, a number of the wires constituting the inner covering member 40C is not limited to eight, and can be arbitrarily determined. The inner covering member 40C is not limited to the multi-thread coil, and may be a single-thread coil formed from one wire, or alternatively may be coated with a hydrophobic resin material, a hydrophilic resin material, or a mixture thereof.

In this way, various configurations may be adopted for the inner covering member 40C. The same effect as in the first and third embodiments can also be exhibited by this guide wire 1C according to the fourth embodiment. In addition, in the guide wire 1C according to the fourth embodiment, inside the covering member 70, the joining agent applied for forming the joint layer 90 enters a gap between each wire of the inner covering member 40C, or the inside of the inner covering member 40C, so that the inner covering member 40C can be joined to the first and second core shafts 10B and 30B.

When a multi-thread coil is used as the inner covering member 40C, the inner covering member 40C becomes more flexible against bending than a pipe-shaped inner covering member, and therefore even if a bending stress is applied to the installation portion CPB, the stress can be easily dispersed by the first and second core shafts 10B and 30B.

### <Fifth Embodiment>

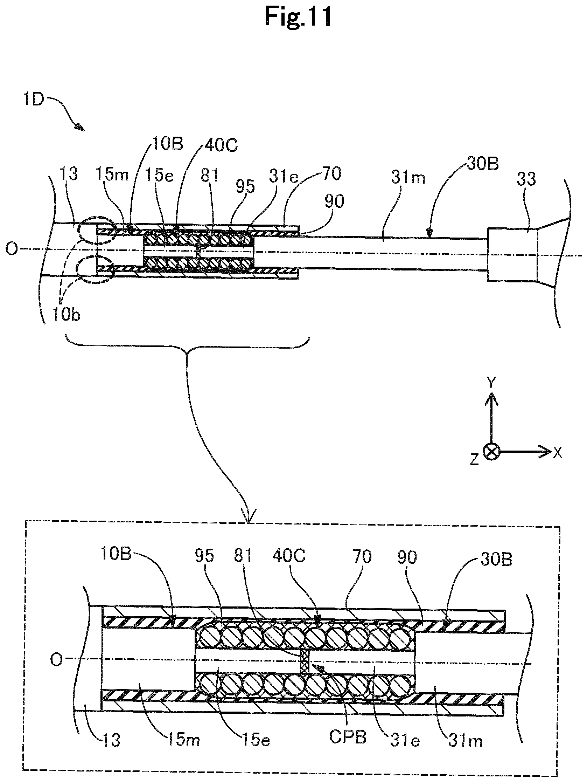

FIG. 11 is a partial sectional view illustrating a vicinity of a contact portion of a guide wire 1D according to the fifth embodiment. The configurations in the upper and lower columns of FIG. 11 are the same as in FIG. 2. The guide wire 1D according to the fifth embodiment includes an inner fixation portion 95 for fixing the inner covering member 40C to the first and second core shafts 10B and 30B in the configuration according to the fourth embodiment. The inner fixation portion 95 is disposed so as to cover the inner covering member 40C, and integrally fixes the first end portion-side small-diameter portion 15e of the first core shaft 10B, the second end portion-side small-diameter portion 31e of the second core shaft 30B, and the inner covering member 40C. The inner fixation portion 95 can be formed from any joining agent, similarly to the distal end-side fixation portion 51. For the inner fixation portion 95 and the distal end-side fixation portion 51, the same joining agent or different joining agents may be used.

In this way, the inner covering member 40C and the covering member 70 may be individually fixed to the first and second core shafts 10B and 30B respectively. The same effect as in the first and third embodiments can also be exhibited by this guide wire 1D according to the fifth embodiment. Furthermore, the same effect on stress distribution as in the fourth embodiment can be exhibited. Additionally, in manufacturing the guide wire 1D, the guide wire 1D according to the fifth embodiment makes it possible to individually perform the step of fixing the inner covering member 40C (forming the inner fixation portion 95) to the first and second core shafts 10B and 30B and the step of fixing a covering member 70C (forming the joint layer 90) to the first and second core shafts 10B and 30B.

### <Sixth Embodiment>

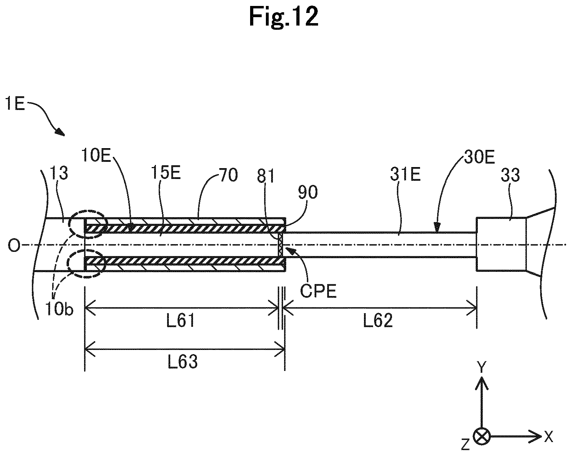

FIG. 12 is a partial sectional view illustrating a vicinity of a contact portion in a guide wire 1E according to the sixth embodiment. In the guide wire 1E according to the sixth embodiment, a length L61 of a first small-diameter portion 15E in the axis line O direction in a first core shaft 10E and a length L62 of a second small-diameter portion 31E in a second core shaft 30E in the axis line O direction are equal (L61=L62). Herein, a substantially same length including an error range is also included in "equal length". Also in the sixth embodiment, in the same way as in the first embodiment, a length L63 of the covering member 70 in the axis line O direction is shorter than a sum of the length L61 of the first small-diameter portion 15E in the axis line O direction and the length L62 of the second small-diameter portion 31E in the axis line O direction (L63<L61+L62). Furthermore, the length L63 of the covering member 70 in the axis line O direction is longer than the length L61 of the first small-diameter portion 15E in the axis line O direction (L63>L61) and longer than the length L62 of the second small-diameter portion 31E in the axis line O direction (L63>L62). Incidentally, the lengths L61, L62, and L63 can be arbitrarily determined as long as they satisfy the aforementioned relationships.

In this way, the lengths of the first and second small-diameter portions 15E and 31E in the axis line O direction in the first and second core shafts 10E and 30E may be substantially equal. The same effect as in the first embodiment can also be exhibited in the sixth embodiment. In addition, the guide wire 1E according to the sixth embodiment facilitates manufacture of the first and second core shafts 10E and 30E because the lengths of the first and second small-diameter portions 15E and 31E in the axis line O direction are equal.

### <Seventh Embodiment>

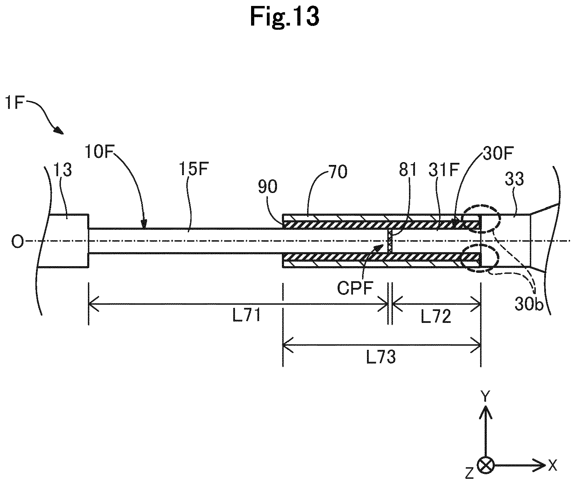

FIG. 13 is a partial sectional view illustrating a vicinity of a contact portion in a guide wire 1F according to the seventh embodiment. In the guide wire 1F according to the seventh embodiment, a dimensional relationship of each length in the axis line O direction of the first and second small-diameter portions 15F and 31F of the first and second core shafts 10F and 30F is reverse to that in the first embodiment. That means, a length L71 in the axis line O direction of the first small-diameter portion 15F in the first core shaft 10F is longer than a length L72 in the axis line O direction of the second small-diameter portion 31F in the second core shaft 30F (L71>L72). In addition, a length L73 in the axis line O direction of the covering member 70 is shorter than a sum of the lengths L71 and L72 (L73<L71+L72), and longer than a shorter one of the lengths of the first and second small-diameter portions 15F and 31F in the axis line O direction (i.e. length L72 of the second small-diameter portion 31F) (L73>L72). Incidentally, the lengths L71, L72, and L73 can be arbitrarily determined as long as they satisfy the aforementioned relationships.

Additionally, in the seventh embodiment, the covering member 70 is disposed such that one end (in the example of the figure, proximal end portion) of the covering member 70 is positioned on a vicinity 30b of a boundary between the second small-diameter portion 31F and the second large-diameter portion 33 in the second core shaft 30F. As in the first embodiment, the covering member 70 covers a contact portion CPF between the first and second core shafts 10F and 30F, and a part of each of the first and second core shafts 10F and 30F adjacent to the contact portion CPF. In the guide wire 1F according to the seventh embodiment, it is sufficient that the "first small-diameter portion 15 of the first core shaft 10" is replaced by "second small-diameter portion 31F of the second core shaft 30F" and the "second small-diameter portion 31 of the second core shaft 30" is replaced by "first small-diameter portion 15F of the first core shaft 10F" in the manufacturing method explained in FIGS. 3A, 3B, 3C, and 3D.

In this way, each length of the first and second small-diameter portions 15F and 31F of the first and second core shafts 10F and 30F in the axis line O direction can be arbitrarily determined, and the side of the second core shaft 30F positioned on the proximal end side of the guide wire 1F may be shortened. The same effect as in the first embodiment can also be exhibited in the seventh embodiment.

### <Eighth Embodiment>

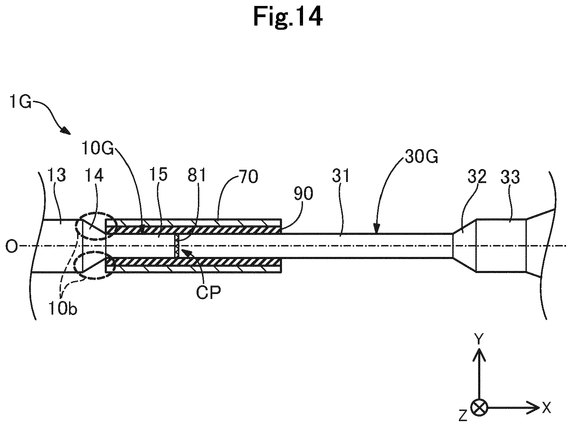

FIG. 14 is a partial sectional view illustrating a vicinity of a contact portion in a guide wire 1G according to the eighth embodiment. In the guide wire 1G according to the eighth embodiment, first and second core shafts 10G and 30G include first and second intermediate portions 14 and 32 respectively. The first intermediate portion 14 is formed between the first large-diameter portion 13 and the first small-diameter portion 15 in the first core shaft 10G. The first intermediate portion 14 has a substantially truncated cone shape with an outer diameter increasing from the proximal end side to the distal end side. The second intermediate portion 32 is formed between the second large-diameter portion 33 and the second small-diameter portion 31 in the second core shaft 30G. The second intermediate portion 32 has a substantially truncated cone shape with an outer diameter decreasing from the proximal end side to the distal end side.

In the configuration including the first and second intermediate portions 14 and 32, the "vicinities 10b and 30b of the boundary" explained in the first embodiment include the first and second intermediate portions 14 and 32. the covering member 70 may be disposed such that the distal end portion of the covering member 70 is positioned on a proximal end portion of the first intermediate portion 14 as illustrated in the figure. In addition, the covering member 70 may be disposed such that the distal end portion of the covering member 70 is positioned on a substantially middle part of the first intermediate portion 14 in the axis line O direction, or on a distal end portion of the first intermediate portion 14. In this way, the configurations of the first and second core shafts 10G and 30G can be arbitrarily modified. The same effect as in the first embodiment can also be exhibited by the guide wire 1G according to the eighth embodiment.

### <Ninth Embodiment>

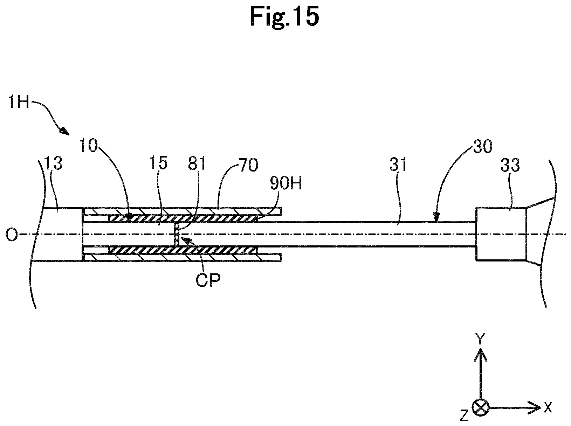

FIG. 15 is a partial sectional view illustrating a vicinity of a contact portion in a guide wire 1H according to the ninth embodiment. The guide wire 1H according to the ninth embodiment includes a joint layer 90H instead of the joint layer 90 in the configuration according to the first embodiment. The joint layer 90H is formed on a part of a gap between the covering member 70 and the first and second core shafts 10 and 30 covered by the covering member 70. Specifically, the joint layer 90H is formed on the substantially middle part but not on the both end portions (distal end portion and proximal end portion) of the covering member 70 in the axis line O direction in the gap. In other words, a void having no joint layer 90H remains inside the both end portions of the covering member 70. In this way, a range where the joint layer 90H is formed can be arbitrarily changed. The same effect as in the first embodiment can also be exhibited by the guide wire 1H according to the ninth embodiment.

### <Tenth Embodiment>

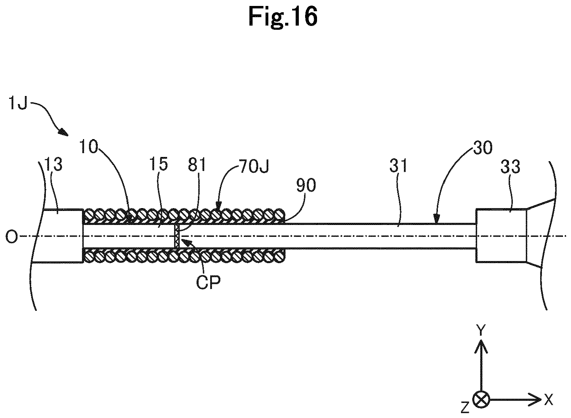

FIG. 16 is a partial sectional view illustrating a vicinity of a contact portion in a guide wire 1J according to the tenth embodiment. The guide wire 1J according to the tenth embodiment includes a covering member 70J having a different configuration from that of the covering member 70 in the configuration according to the first embodiment. The covering member 70J is a substantially hollow cylindrical coil body formed by spirally winding a wire. The covering member 70J may also be a single coil composed of one wire, or a hollow twisted wire coil composed of a twisted wire using a plurality of wires. In addition, the covering member 70J may be the multi-thread coil explained in FIG. 10. The material of the wire constituting the covering member 70J may be made of a material that is the same as or different from that of the wire 21 of the coil body 20. In this way, the shape of the covering member 70J is not limited to the tube shape, and may be arbitrarily changed. The same effect as in the first and fourth embodiments can also be exhibited by the guide wire 1J according to the tenth embodiment.

### <Eleventh Embodiment>

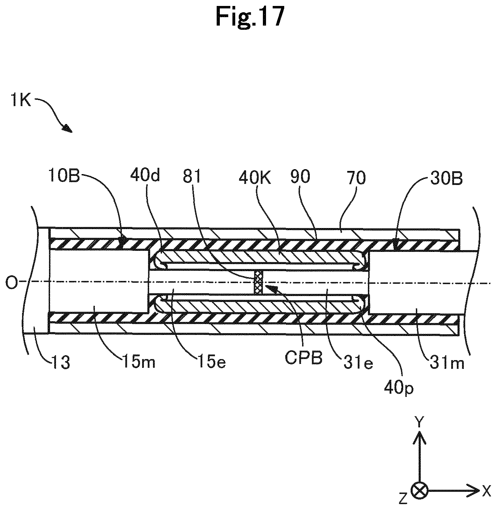

FIG. 17 is an enlarged view illustrating a vicinity of a covering member 70 in a guide wire 1K according to the eleventh embodiment. The guide wire 1K according to the eleventh embodiment includes an inner covering member 40K having a configuration different from that of the inner covering member 40 in the configuration according to the third embodiment. The inner covering member 40K is a metal tubular member formed into a tube shape (substantially hollow cylindrical shape) having a constant outer diameter similarly to the covering member 70. The inner covering member 40K may be made of a material that is the same as or different from that of the covering member 70. The distal end side of the inner covering member 40K is fixed to the first end portion-side small-diameter portion 15e of the first core shaft 10B by caulking a distal end portion 40d. The proximal end side of the inner covering member 40K is fixed to the second end portion-side small-diameter portion 31e of the second core shaft 30B by caulking a proximal end portion 40p. In the example of the figure, there is a void having no joining agent inside the inner covering member 40K. In this way, the inner covering member 40K may be fixed to the first and second core shafts 10B and 30B by a means other than the joining agent. The same effect as in the first and third embodiments can also be exhibited by the guide wire 1K according to the eleventh embodiment.

### <Modification Examples of the Embodiments>

Note that the disclosed embodiments are not limited to the above embodiments, and can be implemented in various aspects without departing from the gist of the disclosed embodiments. For example, the following modifications are also possible.

### [Modification Example 1]

In the aforementioned first to eleventh embodiments, the configurations of the guide wires 1 and 1A to 1H have been described as examples. However, the configuration of the guide wire can be variously modified. For example, the guide wire according to each embodiment has been explained as a medical appliance used for inserting a catheter into a blood vessel, but can also be configured as a guide wire to be inserted into each organ in a human body, such as a lymphatic system, a biliary system, a urinary system, a respiratory system, a digestive system, a secretory gland, and a genital organ. For example, the guide wire may be configured such that the whole of the first and second core shafts (in other words, throughout from the distal end portion of the first core shaft to the proximal end portion of the second core shaft) is covered by the coil body. For example, the guide wire may be productized in a state that the distal end side is previously curved.

### [Modification Example 2]

In the aforementioned first to eleventh embodiments, the configurations of the first and second core shafts 10, 10A, 10B, 10E to 10G, 30, 30A, 30B, and 30E to 30G have been described as examples. However, the configurations of the first core shaft and the second core shaft can be variously modified. For example, the first core shaft does not necessarily include the distal small-diameter portion and the distal decreasing-diameter portion, and the second core shaft does not necessarily include the proximal decreasing-diameter portion or the proximal large-diameter portion. For example, the first core shaft may be made of various materials other than the superelastic material, and the second core shaft may be made of a material less susceptible to plastic deformation than of the first core shaft. The first and second core shafts may be made of the same material. For example, the transverse sectional shape of each portion in the first and second core shafts is not necessarily a substantially circular shape, and various shapes (e.g. a substantially rectangular shape, a substantially elliptical shape, and the like) can be adopted.

### [Modification Example 3]

In the aforementioned first to eleventh embodiments, the configuration of the coil body 20 has been described as an example. However, the configuration of the coil body can be variously modified. For example, the coil body may have a densely-wound structure without gaps between the wires adjacent to each other, or a coarsely-wound structure with gaps between the wires adjacent to each other, or a mixed structure of the densely-wound structure and the coarsely-wound structure. In addition, the coil body may have a resin layer coated with, for example, a hydrophobic resin material, a hydrophilic resin material, or a mixture thereof. For example, a transverse sectional shape of the wire of the coil body is not necessarily the substantially circular shape.

### [Modification Example 4]

In the aforementioned first to eleventh embodiments, the configurations of the inner covering members 40, 40B, 40C, 40D, and 40K have been described as examples. However, the configuration of the inner covering member can be variously modified. For example, the inner covering member may be made of a material other than metal (e.g. resin, or the like). For example, the inner covering member may include a base layer for facilitating adhesion of the joining agent. For example, when the inner covering member is formed into a tube shape, the inner covering member may have a hollow elliptically cylindrical transverse sectional shape. For example, when the inner covering member is a coil body, the wire does not necessarily have a substantially circular transverse sectional shape, and a substantially elliptical shape, a substantially rectangular shape, or the like can be adopted.

### [Modification Example 5]

In the aforementioned first to eleventh embodiments, the configurations of the covering members 70, 70A, 70C, and 70J have been described as examples. However, the configuration of the covering member can be variously modified. For example, the covering member may be made of a material other than metal (e.g. resin or the like). For example, the covering member may be configured so as to have a bending rigidity that is substantially equivalent to or higher than that of the second core shaft, and to have a different elastic modulus from that of the first core shaft.

### [Modification Example 6]

The configurations of the guide wires 1, and 1A to 1K according to the first to eleventh embodiments and the configurations of the guide wires according to the modification examples 1 to 5 may be appropriately combined. For example, in the guide wires 1B to 1D (configuration including the first end portion-side small-diameter portion and the first intermediate small-diameter portion) according to the third to fifth embodiments, the lengths of the first and second small-diameter portions explained in the sixth embodiment (L61=L62), or the lengths of the first and second small-diameter portions explained in the seventh embodiment (L71>L72) may be adopted. For example, in the guide wires 1B to 1D (configuration including the first end portion-side small-diameter portion and the first intermediate small-diameter portion) according to the third to fifth embodiments, the configuration including the first and second intermediate portions explained in the eighth embodiment may be adopted, and the joint layer explained in the ninth embodiment may be adopted.

As described above, the present aspects have been explained based on the embodiments and the modification examples, and the embodiments of the aforementioned aspects are intended to facilitate understanding of the present aspects and not to limit the present aspects. The present aspects can be modified and improved without departing from the gist of the aspects and claims, and the present aspects include equivalents thereof. In addition, if technical characteristics of the present aspects are not explained as essentials in this specification, the technical characteristics can be appropriately deleted.

### DESCRIPTION OF REFERENCE NUMERALS

- 1, 1A to 1K: Guide wire
- 10, 10A, 10B, 10E to 10G: First core shaft
- 11: Distal small-diameter portion
- 12: Distal decreasing-diameter portion
- 13: First large-diameter portion
- 14: First intermediate portion
- 15, 15A, 15E, 15F: First small-diameter portion
- 15e: First end portion-side small-diameter portion
- 15m: First intermediate small-diameter portion
- 20: Coil body
- 21: Wire
- 30, 30A, 30B, 30E to 30G: Second core shaft
- 31, 31A, 31E, 31F: Second small-diameter portion
- 31e: Second end portion-side small-diameter portion
- 31m: Second intermediate small-diameter portion
- 32: Second intermediate portion
- 33: Second large-diameter portion
- 34: Proximal decreasing-diameter portion
- 35: Proximal large-diameter portion
- 40, 40B, 40C, 40D, 40K: Inner covering member
- 41: Wire
- 51: Distal end-side fixation portion
- 52: Proximal end-side fixation portion
- 61: Intermediate fixation portion
- 70, 70A, 70C, 70J: Covering member
- 81: Joining agent
- 90, 90H: Joint layer
- 91, 92, 93: Joint layer
- 95: Inner fixation portion

## Claims

1. A guide wire comprising:
a first core shaft disposed on a distal end side and having a first large-diameter portion and a first small-diameter portion with a diameter smaller than of the first large-diameter portion;
a second core shaft disposed on a proximal end side and having a second large-diameter portion and a second small-diameter portion with a diameter smaller than of the second large-diameter portion;
a covering member that covers a contact portion, where the first small-diameter portion and the second small-diameter portion are arranged oppositely to each other, and at least a part of each of the first small-diameter portion and the second small-diameter portion, the part being adjacent to the contact portion; and
a joint layer that joins the covering member with the first small-diameter portion and the second small-diameter portion inside the covering member, wherein
a length in an axial direction of the covering member is:
shorter than a sum of the length in the axial direction of the first small-diameter portion and the length in the axial direction of the second small-diameter portion; and
longer than a shorter one of the length in the axial direction of the first small-diameter portion and the length in the axial direction of the second small-diameter portion.

2. The guide wire according to claim 1, wherein
the first small-diameter portion has a length in the axial direction shorter than the length in the axial direction of the second small-diameter portion, and a bending rigidity of a material for forming the first core shaft is lower than the bending rigidity of a material for forming the second core shaft.

3. The guide wire according to claim 2, wherein
the first core shaft is made of a superelastic material.

4. The guide wire according to any one of claims 1 to 3, wherein
the covering member is disposed to position one end of the covering member in a vicinity of a boundary between the first large-diameter portion and the first small-diameter portion, and
the joint layer joins at least the contact portion, the first small-diameter portion, and the second small-diameter portion together inside the covering member.

5. The guide wire according to any one of claims 1 to 3, wherein
the covering member is disposed in a vicinity of a center between the first small-diameter portion and the second small-diameter portion joined to each other, and
the joint layer joins at least both end portions of the covering member with the first small-diameter portion and the second small-diameter portion, respectively, inside the covering member.

6. The guide wire according to any one of claims 1 to 5, wherein
the first small-diameter portion includes a first end portion-side small-diameter portion disposed on an end portion side of the first core shaft, and a first intermediate small-diameter portion disposed between the first end portion-side small-diameter portion and the first large-diameter portion and having a diameter larger than of the first end portion-side small-diameter portion,
the second small-diameter portion includes a second end portion-side small-diameter portion disposed on an end portion side of the second core shaft, and a second intermediate small-diameter portion disposed between the second end portion-side small-diameter portion and the second large-diameter portion and having a diameter larger than of the second end portion-side small-diameter portion, and
an inner covering member that covers the contact portion as well as the first end portion-side small-diameter portion and the second end portion-side small-diameter portion, which are adjacent to the contact portion, is further disposed inside the covering member.

7. The guide wire according to any one of claims 1 to 6, wherein
an outer diameter of the covering member is substantially equal to a diameter of the first large-diameter portion.

8. The guide wire according to any one of claims 1 to 7, wherein
the covering member is a tubular member having a tubular shape and made of a superelastic material.

9. A manufacturing method for a guide wire, comprising:
a preparation step of preparing a first core shaft having a first large-diameter portion and a first small-diameter portion with a diameter smaller than of the first large-diameter portion, a second core shaft having a second large-diameter portion and a second small-diameter portion with a diameter smaller than of the second large-diameter portion, and a covering member having a length in an axial direction that is shorter than a sum of the length in the axial direction of the first small-diameter portion and the length in the axial direction of the second small-diameter portion and longer than a shorter one of the length in the axial direction of the first small-diameter portion and the length in the axial direction of the second small-diameter portion;
an introduction step of introducing the covering member from a side of the second small-diameter portion of the second core shaft;
an arrangement step of arranging the first small-diameter portion of the first core shaft and the second small-diameter portion of the second core shaft oppositely to each other;
an application step of applying a joining agent onto a surface of a contact portion, where the first small-diameter portion and the second small-diameter portion are arranged oppositely to each other, and a surface of each of the first small-diameter portion and the second small-diameter portion, the surface being adjacent to the contact portion and being exposed from the covering member; and
a movement step of moving the covering member to positions where the joining agent is applied.

10. A manufacturing method for a guide wire, comprising:
a preparation step of preparing a first core shaft having a first large-diameter portion and a first small-diameter portion with a diameter smaller than of the first large-diameter portion, a second core shaft having a second large-diameter portion and a second small-diameter portion with a diameter smaller than of the second large-diameter portion, and a covering member having a length in an axial direction that is shorter than a sum of the length in the axial direction of the first small-diameter portion and the length in the axial direction of the second small-diameter portion and longer than a shorter one of the length in the axial direction of the first small-diameter portion and the length in the axial direction of the second small-diameter portion;
an introduction step of introducing the covering member from a side of the second small-diameter portion of the second core shaft;
an arrangement step of arranging the first small-diameter portion of the first core shaft and the second small-diameter portion of the second core shaft oppositely to each other;
a first application step of applying a joining agent onto a surface of the first small-diameter portion that is exposed from the covering member;
a first movement step of moving the covering member to a position where the joining agent is applied;
a second application step of applying the joining agent onto a surface of the second small-diameter portion that is exposed from the covering member; and
a second movement step of moving the covering member in a vicinity of a center between the first small-diameter portion and the second small-diameter portion.
